# EUROPEAN PATENT APPLICATION

(11) **EP 1 101 485 A1**
(43) Date of publication of application: **23.05.2001**
(21) Application number: 00123524.1
(22) Date of filing: 27.10.2000
(51) Int. Cl.: A61K 7/06, A61K 7/48

(54) **Viscosity stabilized compositions comprising a zeolite and a solid fatty alcohol**

(30) Priority: 18.11.1999 US 442719
(71) Applicant: AVON PRODUCTS, INC., New York, NY 10020-1196 (US)
(72) Inventor: Garrison, Mark S., Suffern, NY 10901 (US)
(74) Representative: Dr. Weitzel & Partner

(57) **Abstract**

There is disclosed a viscosity stabilized composition in the form of an oil-in-water emulsion, which comprises effective amounts of a zeolite and a solid fatty alcohol.

## Description

### BACKGROUND OF THE INVENTION

### 1. Field of the Invention

The present invention relates to emulsified compositions having zeolite and a solid fatty alcohol. These ingredients have been found highly effective to improve the stabilization of the compositions against viscosity loss at elevated or higher temperatures.

### 2. Description of the Prior Art

Skin treatment compositions are extensively described in the art. Emulsified skin treatment compositions are disclosed in U.S. Patent No. 4,362,715 to Strianse et al., issued on December 7, 1982. It discloses gels formed by combining a carboxyvinyl polymer and a zeolite. The creamy gel product is said to be useful in formulating cosmetic compositions including emulsions and provides desirable rheology to such compositions.

### SUMMARY OF THE INVENTION

It is an object of the present invention to provide an emulsified composition.

It is another object of the present invention to provide such a composition that has been found highly effective in stabilizing the composition against viscosity loss at elevated or higher temperatures without a corresponding increase in viscosity at room temperature.

It is a further object of the present invention to provide a method of stabilizing the viscosity of a composition using zeolite and a normally solid fatty alcohol.

These and other objects of the present invention will be appreciated by the present invention that is a viscosity stabilized composition in the form of an oil-in-water emulsion. The composition includes an amount effective to stabilize the viscosity of the composition at high temperatures in which the composition comprises a zeolite and a normally solid fatty alcohol. Preferably, the zeolite is an aluminosilicate zeolite.

### DESCRIPTION OF THE INVENTION

The present invention is based on the discovery that zeolite with a normally solid fatty alcohol functions as a highly effective high temperature viscosity stabilizer in oil-in-water emulsions. The oil-in-water emulsions include, but are not limited to, cosmetic and/or pharmaceutical skin treatment, skin care, hair care, personal care and color compositions (hereinafter called "composition"). The combination has been tested and shown to provide to such emulsions viscosity stability at elevated temperatures, i.e temperatures up to at least about 100 degrees F. to about 120 degrees F. This is accomplished with virtually no change of viscosity at room temperature. Viscosity stability at such high temperatures is the meaning of stability as defined in this application. Also, the compositions of the present invention are particularly useful for stabilizing the viscosity of emulsions formulated at a low pH level, i.e., about 2 to about 5, especially at about 3 to about 4. Furthermore, the zeolite does not change the aesthetics of the composition. Significantly, most commercially available thickeners are less effective (1) at high temperatures, i.e., temperatures about 90 to about 100 degrees F. (26.6 to 37.8 degrees C.), and (2) at low pHs.

Preferably, the zeolite used in the practice of the present invention can be a natural or synthetic zeolite. More preferably, the zeolite is a lipophilic zeolite and, most preferably, an aluminosilicate zeolite. The most preferred zeolite is a sodium aluminosilicate, that is lipophilic. In addition, it is microporous. The aluminosilicate molecular sieves comprise the large class of well-known zeolites. These high-silica molecular sieves are either commercially available or are prepared by methods, well-known in the art, involving direct hydrothermal synthesis or involving certain types of crystal lattice dealuminations. A comprehensive review article by E. M. Flanigen concerning both "high" Si/Al zeolites and silica molecular sieves is published in "Proc. 5th Int. Conf. Zeolites, Naples, 1980", L. V. C. Rees, Ed., Heyden, London, pp. 760-780. This article is incorporated herein by reference.

A suitable class of zeolites is characterized as "intermediate" silicate/aluminate zeolites. The intermediate zeolites are characterized by silica to alumina molar ratios of less than about 10, such as from about 2 to about 10.

The more preferred zeolites, sodium aluminosilicate zeolites, have silica to alumina molar ratios of at least 35 and preferably from 200 to 500, an average particle size of 3.5 to 6.5 microns, and a pore size of at least 5.5 angstroms. One such zeolite is sold under the name "ABSCENTS" by UOP, Molecular Sieve Division that apparently is disclosed in U.S. Patent No. 4,795,482. Many of the synthetic zeolites, which are prepared using organic templating agents, are readily produced in a highly siliceous form. In many instances, the reaction mixtures can be especially free of aluminum-containing reagents. These zeolites are markedly organophilic and include ZSM-5 (U.S. Patent No. 3,702,886); ZSM-11 (U.S. Patent No. 3,709,979); ZSM-23 (U.S. Patent No. 4,076,842); ZSM-35 (U.S. Patent No. 4,016,245); and ZSM-38 (U.S. Patent No. 4,046,859) to name only a few.

The zeolite in the present composition is in an amount about 0.1 wt% to about 10 wt%, preferably about 0.5 wt% to about 5.0 wt%, and most preferably about 1.0 wt% to about 3.0 wt%.

The second ingredient in the present composition is a normally solid fatty alcohol. Suitable solid fatty alcohols in the context of the present invention are understood to be any fatty alcohol that contains solid fatty alcohols at room temperature (namely 77 degrees F). Typical examples of such solid fatty alcohols are lauryl, myristyl, cetyl, stearyl, hydroxystearyl, dihydroxystearyl and behenyl alcohols and mixtures thereof. These alcohols are obtained, for example, in the high-pressure hydrogenation of technical methyl esters based on fats and oils or aldehydes from Roelen's oxosynthesis and which accumulate as monomer fraction in the dimerization of unsaturated fatty alcohols.

In the present invention, the preferred solid fatty alcohol is a fatty alcohol containing cetyl alcohol, stearyl alcohol, or mixtures thereof.

The solid fatty alcohol is in the present emulsions in an amount about 0.1 wt% to about 10 wt%, preferably about 4.0 wt% to about 5.5 wt%, and most preferably about 4.5 wt% to about 5.0 wt% of the total weight of the composition.

It is preferred that a non-ionic emulsifier be in the composition of the present invention. The non-ionic emulsifier can be in a mixture with the solid fatty alcohol. If the mixture is used, then the solid fatty alcohol is used in an amount preferably about 3.5 wt% to about 4.5 wt%, and most preferably about 3.5 wt%.

Suitable non-ionic emulsifiers in the mixture include fatty alcohol ethoxylates. For example, one such non-ionic emulsifier is Ceteareth 20 (polyethylene glycol 20 cetyl/stearyl alcohol).

Preferred examples of such a mixture are solid fatty alcohols and non-ionic emulsifiers that contain identical alkyl radicals. Such mixtures include, for example, mixtures of cetearyl oligoglucoside and cetearyl alcohol. For this mixture, there is about 60 wt% to about 80 wt% of solid fatty alcohol and about 20 wt% to about 40 wt% of glucosides. An example of such a preferred solid fatty alcohol/fatty alcohol glucoside mixture is the product marketed under the name "Montanov 68" by the company Seppic. The mixture essentially consists of the combination of approximately 80% of cetyl/stearyl alcohol (C₁₆-C₁₈) and approximately 20% of cetearyl glucoside.

The mixture of solid fatty alcohol/non-ionic emulsifier is present in an amount about 0.5 wt% to about 15 wt%, preferably about 1.0 wt% to about 5 wt%, and most preferably about 1.00 wt% to about 2.25 wt%, of the total weight composition.

The present composition also provides for the use of a second normally solid fatty alcohol in addition to the first solid fatty alcohol. The second solid fatty alcohol can be in the mixture or in addition to the mixture.

The present emulsions may include other ingredients. For example, water may comprise about 40 wt% to about 80 wt%, preferably about 40 wt% to about 55 wt%, and most preferably about 45 wt% to about 50 wt%, of the total weight of the composition of the present invention.

In addition to water, suitable inorganic thickeners, which are not a zeolite, can be used in the present invention. Such inorganic thickeners include clays, such as bentonite, hectorite, kaolin, montmorillonite, silica; and metal silicates, such as calcium silicate, aluminum silicate and preferably magnesium aluminum silicate. They are employed in amounts about 0.01 wt% to about 10 wt%, preferably about 0.2 wt% to about 2.0 wt%, and most preferably about 0.4 wt%, of the total weight of the composition.

The compositions of the present invention will typically contain at least one or more other ingredients. For example, the skin treatment formulations of the present invention will typically contain conventional cosmetic ingredients necessary in formulating a desirable product.

The composition may have an organic thickener to ensure that it has the proper viscosity when applied to the skin. Examples of such thickeners, which may be used, include hydroxyethyl cellulose, carboxymethyl cellulose, xanthan gum and polyacrylamide (Seppi Gel 305). The amount of such organic thickener is typically in the range about 0.01 wt% to about 10 wt%, preferably about 0.5 wt% to 3.5 wt%, and most preferably about 1.5 wt%, of the total weight of the composition.

The compositions of the present invention may contain one or more sunscreens or UV (ultraviolet) absorbing agents. Examples of such sunscreens in preferred amounts are:

| Sunscreen | Percentage (wt%) |
|---|---|
| oxybenzone | 2 to 10 |
| sulsiobenzone | 5 to 10 |
| dioxybenzone | 1 to 3 |
| menthyl anthranilate | 3 to 6 |
| para aminobenzoic acid (PABA) | 5 to 15 |
| dea methoxycinnamate | 8 to 10 |
| octocrylene | 7 to 10 |
| octyl methoxycinnamate * | 2 to 10 |
| octyl salicylate | 3 to 5 |
| homomenthyl salicylate | 4 to 15 |
| octyl dimenthyl PABA | 1.4 to 5 |
| tea salicylate | 5 to 12 |
| titanium dioxide | 2 to 25 |
| zinc oxide | 2 to 25 |
| butylmethoxy | 0.1 to 5 |
| dibenzoylmethane ** | |
| 4-methyl benzilidene camphor | 0.1 to 6 |
| octyl triazone | 0.1 to 10 |
| terephthalydidene dicamphor | 0.1 to 5 |
| sulfonic acid and salts thereof *** | |
| ethyl PABA | 1 to 10 |
| 2-(2' hydroxy-5' -methylphenyl) benzotriazole **** | 0.5 to 10 |
| methylene bis-benzotriazolyl tetramethylbutylphenol ***** | 1 to 10 |
| bis-octoxyphenol methoxphenyl triazine ****** | 1 to 10 |

| | |
|---|---|
| * The term "octyl methoxycinnamate" and "ethylhexyl methoxycinnamate" are used interchangeably. | |
| ** A non-limiting example of butylmethoxy dibenzoylmethane is available from Givaudan under the tradename "PARSOL 1789". | |
| *** A non-limiting example of terephthalydidene dicamphor sulfonic acid and salts thereof is available from L'Oreal under the tradename "MEXORYL SX". | |
| **** A non-limiting example of 2-(2'hydroxy-5'-methylphenyl) benzotriazole is available from Ciba-Geigy under the tradename "TINUVIN P". | |
| ***** A non-limiting example of methylene bis-benzotriazolyltetramethylbutylphenol is available from Ciba-Geigy under the tradename "TINOSORB-M". | |
| ****** A non-limiting example of bis-octoxyphenol methoxphenyl triazine is available from Ciba-Geigy under the tradename "TINOSORB-S". | |

The composition may contain an antioxidant, such as gamma oryzanol (a ferulic acid ester of cycloartenol), mixed tocopherol (a mixture of isomers of Vitamin E), ascorbyl monopalmitate, ascorbyl phospheryl cholesterol, butylated hydroxy toluene, tomato extract (a natural extract which contains lycopene), or rosemary extract (Rosmarinus officinalis). Preferably, the antioxidant is present in an amount about 0.01 wt% to about 5.0 wt% of the total weight of the composition.

The compositions of the present invention may also contain about 1 wt% to about 20 wt% of a humectant, preferably about 1 wt% to about 5 wt%. The most preferred humectant is glycerin and in an amount about 3 wt%. Other suitable humectants include sorbitol, sodium 2-pyrrolidonecarboxylate, collagen, ethoxylate or propoxylate of glucose, polyethylene glycol (for example, Carbowax 400), propylene glycol and butylene glycol.

One or more emollients may also be present in the present composition in an amount about 8 wt% to about 20 wt%. Suitable emollients or oleaginous materials include mineral oil, petrolatum, glyceryl monooleate, isopropyl palmitate, avocado oil, squalane, octyl palmitate, cocoa butter, sesame oil, propylene glycol dicaprylate/dicaprate, C₁₂-C₁₅ alcohol esters of benzoic acid, dicaprylyl maleate, isopropyl myristate, diisopropyl dimerate (that is, the diester of isopropyl alcohol and dimer acid), dimethicone, stearoxydimethicone, octyl dodecanol, octyl dodecyl neopentanoate, neopentyl glycol dioctanoate, and hydrogenated polydecene. The preferred emollients are petrolatum, octyl dodecanol, octyl dodecyl neopentanoate, neopentyl glycol dioctanoate, and hydrogenated polydecene, which also function as occlusivity agents.

The present composition may also include one or more insect repellents. Such insect repellents include oil of citronella, Deet, and ethyl 3-(N-butylacetamino) propionate. The preferred insect repellent is ethyl 3-butylacetamino) propionate (sold under the commercial name Merck IR3535, by Merck Corporation). This preferred insect repellent is preferably present in an amount about 5 wt% to about 20 wt% of the total weight of the composition.

One or more exfoliants can be included in the present composition. Such exfoliants include one or more alpha hydroxy acids, beta hydroxy acids, oxa acids, oxa diacids, or mixtures thereof. If the exfoliant is an alpha hydroxy acid, it is present in an amount about 2 wt% to about 10 wt%, preferably about 5 wt%. If the exfoliant is either oxa acid or oxa diacid or a combination of both, it is present in an amount about 5 wt% to about 10 wt%.

The present composition may include one or more bodying or thickening agents, such as stearic acid and glyceryl monostearate, and pH adjusters, such as ammonium hydroxide, each in an amount about 1 wt% to about 10 wt% and preferably about 1 wt% to about 5 wt%; one or more coloring agents or pigments, such as iron oxides and organic dyes, that total about 0.001 wt% to about 1 wt%; one or more preservatives, such as paraben including ethyl paraben, methyl paraben, propyl paraben, butyl paraben, 2-phenoxyethanol, disodium EDTA (ethylenediamine tetraacetic acid), Glydant (dimethyldimethoyl hydantoin), benzyl alcohol, and imidazolidinyl urea, that total about 1 wt% to about 3 wt%; and one or more skin protecting agents, such as dimethyl polysiloxane or panthenol, in an amount about 0.1 wt% to about 5 wt%.

Numerous other special purpose additives may be added to the compositions of the present invention in minor amounts, namely less than about 2.0 wt%. These include an odor remover, such as zinc ricinoleate. It may also include one or more anti-aging agents, deodorizers, fragrances, alcohols, liquid fatty alcohols, masking agents, and skin healing agents.

The compositions of this invention are prepared using techniques well known in the emulsion art, such as by first adding any water soluble ingredients to the aqueous phase, heating the aqueous phase to about 50 to about 90° C., then dispersing into the aqueous phase the emulsifiers and oleaginous components with stirring and allowing the composition to cool to room temperature whereby a stable emulsion is formed.

The present invention is illustrated by the following example of a skin care and treatment composition (percentages are by weight):

### EXAMPLE

Emulsion 1: a stable oil-in-water emulsion skin treatment composition was prepared which contained 44% water, 0.4% of magnesium aluminum silicate, 0.99% of a crystalline sodium aluminosilicate zeolite having an average particle size of 3.5 to 6.5 microns, 1.25% of a mixture composed of 20% cetearyl glucoside and 80% cetearyl alcohol, and the balance comprised several conventional skin treatment ingredients including one or more emulsifiers, antioxidants, alpha hydroxy acids, emollients, humectants, sunscreens, skin protectants, pH adjusters, preservatives, and organic thickeners. The viscosity of Emulsion 1 at 100° F. was TC 30 (measured using the TC spindle at 4 rpm of a Brookfield viscometer, with the spindle factor for TC to centipoise being 2,500), and at room temperature TC 38. Without the zeolite, Emulsion 1 had a viscosity of TC 14 at 100° F (35,000 centipoise), and at room temperature a TC 41.

Emulsion 2 was substantially the same as Emulsion 1 except it had 47% water, 0.3% of magnesium aluminum silicate, and 0.99% of the mixture composed of 20% cetearyl glucoside and 80% cetearyl alcohol. It had a viscosity of TC 20 at 100° F., and at room temperature a viscosity of TC 43, and a viscosity of TC 11 at 100° F. without the zeolite, and at room temperature a viscosity of TC 46.

Significantly, the room temperature viscosity of all compositions for both Emulsions did not change.

Thus, the composition is thicker when hot if zeolite is used in the composition, than if zeolite is not used.

The present invention having been described with particular reference to the preferred forms thereof, it will be obvious that various changes and modifications may be made herein without departing from the spirit and scope of the present invention as defined by the appended claims.

## Claims

1. A method of stabilizing the viscosity of a composition at high temperatures comprising adding to the composition a zeolite and a solid fatty alcohol.

2. The method of claim 1, wherein the composition further comprises a non-ionic emulsifier.

3. The method of claim 2, wherein the emulsifier is a fatty alcohol glucoside.

4. The method of claim 1, wherein said zeolite is present in an amount about 0.1 wt% to about 10 wt% of the total weight of the composition.

5. The method of claim 1, wherein said solid fatty alcohol is present in an amount about 0.1 wt% to about 10 wt% of the total weight of the composition.

6. A method of stabilizing the viscosity of a composition at high temperatures comprising adding to the composition a zeolite, and a mixture of a solid fatty alcohol and a fatty alcohol glucoside.

7. A viscosity stable composition in the form of an oil-in-water emulsion, which composition comprises:
(a) a zeolite; and
(b) a solid fatty alcohol,
wherein an amount of said zeolite and said fatty alcohol is effective to stabilize the viscosity of the composition at high temperatures, while the viscosity of the composition remains virtually the same at room temperature.

8. The composition of claim 7, wherein said zeolite is present in an amount about 0.1 wt% to about 10 wt% of the total weight of the composition.

9. The composition of claim 7, wherein said zeolite is an aluminosilicate zeolite.

10. The composition of claim 9, wherein said zeolite is a sodium aluminosilicate having a silica to alumina molar ratio of at least 35 and an average particle size of 3.5 to 6.5 microns.

11. The composition of claim 7, wherein said solid fatty alcohol is present in an amount about 0.1 wt% to about 10 wt% of the total weight of the composition.

12. The composition of claim 7, further comprising a non-ionic emulsifier.

13. The composition of claim 12, wherein said non-ionic emulsifier is a fatty alcohol glucoside.

14. The composition of claim 13, wherein said solid fatty alcohol is cetearyl, and said fatty alcohol glucoside is a cetearyl glucoside.

15. The composition of claim 7, further comprising a second solid fatty alcohol.

16. The composition of claim 15, further comprising a non-ionic emulsifier.

17. The composition of claim 7, further comprising an inorganic thickener other than a zeolite.

18. The composition of claim 17, wherein said inorganic thickener is present in an amount about 0.01 wt% to about 10 wt% of the total weight of the composition.

19. The composition of claim 17, wherein said inorganic thickener is selected from the group consisting of metal silicates, clays, and mixtures thereof.

20. The composition of claim 7, further comprising at least one or more ingredients selected from the group consisting of antioxidant, anti-aging agent, bodying agent, coloring agent, emollient, exfollient, fragrance, humectant, alcohol, liquid fatty alcohol, insect repellent, organic thickener, pH adjuster, preservative, skin healing agent, skin protecting agent, and sunscreen.
